# EUROPÄISCHE PATENTSCHRIFT

(11) **EP 1 367 170 B1**
(45) Veröffentlichungstag und Bekanntmachung des Hinweises auf die Patenterteilung: **29.10.2008**
(21) Anmeldenummer: 02012037.4
(22) Anmeldetag: 31.05.2002
(51) Int. Cl.: D06H 3/08, G01P 3/80, G01P 3/68, G01N 33/36, G01N 21/89, D01G 31/00

(54) **Doppelsensor**
Dual sensor
Détecteur à deux capteurs

(43) Veröffentlichungstag der Anmeldung: 03.12.2003
(73) Patentinhaber: Hergeth, Hubert A., 6300 Zug (CH)
(72) Erfinder: Hergeth, Hubert A., 6300 Zug (CH)

(56) Entgegenhaltungen:
- EP-A- 0 409 318
- CH-A- 654 608
- DE-A- 4 340 165
- DE-A- 4 441 864
- US-A- 6 118 132
- PATENT ABSTRACTS OF JAPAN vol. 013, no. 422 (P-933), 20. September 1989 (1989-09-20) -& JP 01 153945 A (KITA DENSHI:KK), 16. Juni 1989 (1989-06-16)
- DATABASE WPI Section EI, Week 199226 Derwent Publications Ltd., London, GB; Class S02, AN 1992-215528 XP002228295 -& SU 1 681 243 A (SYNTH FIBRES PROCESSING RES INST), 30. September 1991 (1991-09-30)

## Beschreibung

Die Anmeldung betrifft einen Sensor zum Überwachen von bewegten Materialbahnen z.B. Textil. In dem Sensorgehäuse 1), das sich über die Breite der Textilbahn 7) erstreckt, oder mehrere Sensorgehäuse, die die Bahnbreite abdecken, befindet sich eine Reihe von Licht empfindlichen Sensoren 4), mindestens eine stabförmige Linse 3) und eine Beleuchtung 5). Für viele Anwedungen ist es notwendig, die Geschwindigkeit der Materialbahnen zu erfassen. Auf konventionellem Wege werden diese Geschwindigkeiten durch Kontakte der Bahn mit Drehgebern erfasst. Es ist auch ein Verfahren bekannt geworden, bei dem bewegte Bazhnen mittels Laserstrahl abgestastet werden und das reflektierende Licht an 2 versetzt angeordnete Empfänger gelangt. Die von den beiden Empföngern empfangenen Lichtsignale werden nach Frequenz und Intensität analysiert. Es wird untersucht, ob eine vom ersten Empfänger aufgenommene Sequenz ähnlich, zeitlich leicht versetzt vom zweiten Empfänger aufgenommen wird. Aus dem zeitlichen Versatz und dem bekannten Abstand der Empfänger wird die Geschwindigkeit der Bahn berechnet. Die US-6118132 zeigt ein Verfahren zur Geschwindigkeitsermittlung mittels 2 Photosensorenanordnungen. Die DE 4340165 zeigt ein System zur Inspektion von Faserflocken. Die CH 654608 zeigt einen Sensor zur Feststellung von Fehlern in Fasern. Aufgabe der Erfindung ist es, einen Sensor zu schaffen, der sowohl das Vlies optisch auf Defekte inspizieren kann als auch die Geschwindigkeit der Bahn zu messen. Erfindungsgemäß geschieht dies dadurch, daß in einem Gehäuse 1) sowohl eine Reihe von fotosensitigen Sensoren 4), eine Beleuchtung 5) und mindestens eine stabförmige Linse 3) enthalten sind und 90° zur Längenachse des Sensorgehäuses von der ersten Sensorzeile 4) in einem gewissen Abstand mindestens ein weiterer fotosensitiver Sensor 6) angebracht ist. Die Signale des zweiten Sensors 6) und die Signales des ihm in der ersten Reihe gegenüberliegenden Sensors 4) werden vom Computer zur Geschwindigkeitsmessung ausgewertet. Der zeitliche Signalverlauf beider gegenüberliegender Sensorpunkte wird aufgezeichnet und bei identischem Verlauf aus dem zeitlichen Versatz der Signalverläufe die Geschwindigkeit berechnet. Der zweite Sensor 6) kann an beiliebiger Stelle der ersten Sensorzeile geenüber liegen. Es ist auch denkbar, einen ersten Sensor für ein erstes Signal und einen zweiten Sensor für das zweite Signal unabhängig von der Sensorzeile im Gehäuse des Bahnsensors zu plazieren, dies ist aber umständlicher.

Die Skizze a) zeigt eine typische Anordnung in einem Gehäuse (1) vorzugsweise aus extrudiertem Aluminium sind eine erste Sensorzeile (4), die sich über einen Großteil der Länge des Profils.erstreckt, angebracht. Die Sensoren sehen über eine Stablinse (3) durch eine Glasscheibe (2) auf eine Faserbahn (7). Die Faserbahn wird durch eine Reihe von LED's (5), die sich über einen Großteil der Länge des Gehäuses erstreckt, beleuchtet. Mindestens ein Sensor (6) ist gegenüber der ersten Sensorzeile in einem Abstand (s) angebracht und betrachtet über eine Linse (3) die Faserbahn. Die beiden Sensoren betrachten Licht, da von einer gemeinsamen Beleuchtung (5) auf die Fahrbahn geworfen wird.

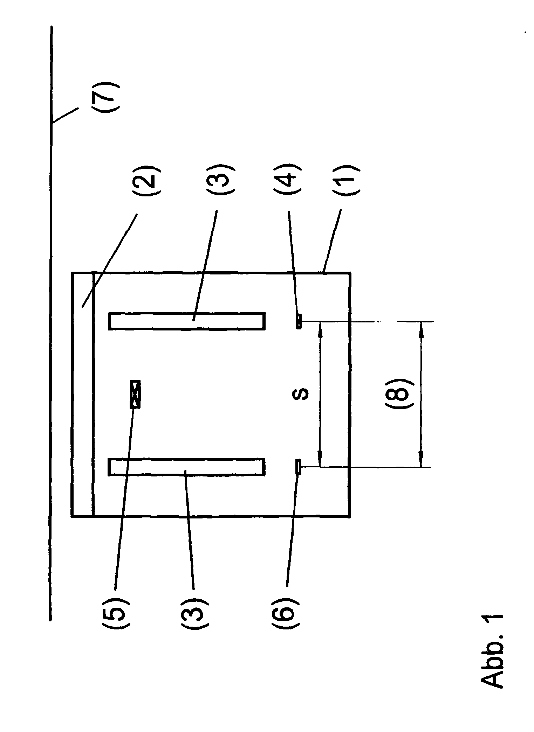

## Patentansprüche

1. Sensor zum Kontrollieren von Materialbahnen und Messen der Geschwindigkeit **dadurch gekennzeichnet, daß** in einem Sensorgehäuse (1) sowohl eine Reihe von Fotosensoren (4) zur Kontrolle der Materialbahnen als auch versetzt zu dieser mindestens ein weiterer Fotosensor (6) in einem Abstand (s) angebracht ist, und die Sensorreihen (4) und der versetzte Sensor (6) mit dem gleichen Computer verbindbar sind.

2. Sensor nach Anspruch 1 **dadurch gekennzeichnet, daß** die Sensorreihe (4) und weitere Sensoren (6) durch eine gemeinsame Glasscheibe (2) die Materialbahn sehen.

3. Sensor nach einem der Ansprüche 1 und 2 **dadurch gekennzeichnet, daß** die Sensorreihe (4) und der versetzt angeordnete Sensor (6) oder Sensoren Licht empfangen, das von der gleichen Lichtquelle(5) ausgesandt wird.

4. Sensor nach einem der Ansprüche 1 bis 3 **dadurch gekennzeichnet, daß** der Abstand (s) zwischen der Sensorreihe und dem versetzt angeordneten Sensor (6) oder Sensoren mindestens 10 mm beträgt.

## Claims

1. Sensor to control width and to control the speed **characterized by** the fact that in a sensor housing (1) an array of photosensors (4) to control the width and at least an additional photosensor (6) that is arranged offset of the first are housed. Both being connected to the same computer.

2. Sensor according to claim 1 **characterized by** the sensor array (4) and the additional sensor (6) controling the width through the same glass window.

3. Sensor according to claim 1 and 2 **characterized by** the sensor array (4) and the offset sensor (6) or sensors receiving reflected light that is emitted by the same light source (5).

4. Sensor according to one of the claims 1- 3 **characterized by** the distance between the sensor array (4) and the offset sensor (6) being at least 10 mm.

## Revendications

1. capteur pour contrôler des voies de matériel et pour mesurer la vitesse **caractérisé par le fait que** dans un boîtier (1) il γ a aussi bien une rangée des capteurs photos pour le contrôle des voies de matériel que transposé à celle-ci au moins un capteur photo (6)de plus dans une distance (s) et que la rangée de capteurs (4) et le capteur transposé sont connectés par le même ordinateur.

2. capteur par titre 1 **caractérisé par le fait que** les rangées de capteurs (4) et les capteurs ultérieurs voient par une vitre commune les voies du matériel.

3. capteur par un des titres 1 et 2 **caractérisé par le fait que** la rangée des capteurs (4) et les capteurs transposés (6) ou senseurs reçoivent de la lumière qui est émise de la même source luminescente (5).

4. capteur par un des titres 1 à 3 **caractérisé par le fait que** la distance (s) entre la rangée des capteurs et le capteur transposé (6) ou les capteurs fait au moins 10 mm.
